Europäisches Patentamt

European Patent Office

Office européen des brevets

(19)

(11) Veröffentlichungsnummer: **0 246 471**

**A1**

## (12) EUROPÄISCHE PATENTANMELDUNG

(21) Anmeldenummer: **87106230.3**

(51) Int. Cl.⁴: **C07C 143/10**

(22) Anmeldetag: **29.04.87**

(30) Priorität: **17.05.86 DE 3616843**

(43) Veröffentlichungstag der Anmeldung:
**25.11.87 Patentblatt 87/48**

(84) Benannte Vertragsstaaten:
**AT BE CH DE ES FR GB GR IT LI LU NL SE**

(71) Anmelder: **AKZO N.V.**
**Velperweg 76**
**NL-6824 BM Arnhem(NL)**

(72) Erfinder: **Bunzel, Wolfgang, Dr.**
**Trockener Weiher 85**
**D-5190 Stolberg(DE)**
Erfinder: **Jansen, Franz**
**Lucherberger Strasse 30**
**D-5176 Inden-Pier(DE)**

(74) Vertreter: **Fett, Günter**
**Akzo GmbH Kasinostrasse 19-23**
**D-5600 Wuppertal 1(DE)**

(54) **Oberflächenaktive Kondensationsprodukte.**

(57) Oberflächenaktive Kondensationsprodukte durch Verestern von Carbonsäuren mit Salzen von Hydroxyalkansulfonsäuren werden hergestellt, indem man Carbonsäuren wie Kokosfettsäuren mit einem Salz der allgemeinen Formel $HO\text{-}(CH_2)_n\text{ -}SO_3X$, wobei n Werte von 2 bis 4 bedeuten und X ein Alkalimetall oder $NH_4$ bedeutet, in Gegenwart eines Konsistenzreglers verestert. Als Konsistenzregler werden flüssige, halbfeste oder feste Paraffine verwendet. Diese Paraffine sind hervorragende Prozeßhilfsmittel bei der Herstellung der Kondensationsprodukte; darüber hinaus können sie im Kondensationsprodukt verbleiben, da bei Syndet-Stücken, welche man aus den Kondensationsprodukten herstellen kann, Paraffingehalte gewünscht werden. Von Vorteil ist ferner, daß bei der Synthese der Kondensationsprodukte der Gehalt an freien Säuren beachtlich herabgesetzt werden kann.

## Oberflächenaktive Kondensationsprodukte

Die Erfindung betrifft ein Verfahren zur Herstellung von oberflächenaktiven Kondensationsprodukten durch Verestern von Carbonsäuren mit Salzen von Hydroxyalkansulfonsäuren.

Die Herstellung derartiger Kondensationsprodukte ist bereits seit langem bekannt. So wird in dem Buch von August Chwala, Textilhilfsmittel, Ihre Chemie, Kolloid-Chemie und Anwendung Verlag J. Springer, Wien, 1939, auf den Seiten 173 bis 174 ein solches Verfahren beschrieben. Dabei wird Ölsäure oder Ölsäurechlorid mit Hydroxyäthansulfonsaurem Natrium verestert. Der dabei entstehende Ester der Ölsäure und des Natrium hydroxyäthansulfonats (Acylisäthionat), auch Igepon genannt, ist oberflächenaktiv und hat sich z.B. als Waschmittel für Proteinfasern und Kunstfasern aus regenerierter Cellulose bewährt.

Die Bedeutung derartiger Kondensationsprodukte hat im Laufe der Zeit zugenommen, und so gibt es zahlreiche Hinweise auf derartige Herstellungsverfahren, sei es in der Patentliteratur oder in Fachzeitschriften.

So wird z.B. in der US-PS 2 635 103 ein Verfahren beschrieben, bei der ein Fettsäuregemisch, in dem der Hauptbestandteil Laurinsäure ist, mit 2-hydroxyäthansulfonsaurem Natrium umgesetzt wird. Die US-PS 2 857 370 lehrt die Umsetzung einer Reihe von Carbonsäuren mit hydroxyalkansulfonsauren Salzen in Gegenwart eines borhaltigen Katalysators. In der europäischen Patentanmeldung Nr. 0067624 wird ein Verfahren zur Herstellung derartiger Kondensationsprodukte beschrieben, bei dem 2-hydroxyalkansulfonsaures Alkali mit einer Fettsäure verestert wird,wobei ein Veresterungskatalysator und zusätzlich ein Alkalimetallhydroxid oder ein Akalimetallsalz einer Carbonsäure eingesetzt wird. Dadurch soll einer Verfärbung des Reaktionsproduktes vorgebeugt werden.

Es hat sich gezeigt, daß es zweckmäßig ist, bei dieser Reaktion für eine gute Homogenisierung der Reaktionsmischung zu sorgen, d.h. es muß während der Umsetzung ständig gerührt werden. Da die Reaktion im allgemeinen einige Stunden dauert und die Viskosität mit zunehmender Veresterung stark ansteigt, ist ein verhältnismäßig hoher Energieaufwand für das Homogenisieren erforderlich. Dies ist besonders gegen Ende der Reaktion ein Problem, wenn der Gehalt an Kondensationsprodukt nahezu seinen Endwert erreicht hat und überschüssige Carbonsäure weitgehend abdestilliert wird. Daher ist es notwendig, entsprechend robuste Rührer einzusetzen, die in der Lage sind, die hochviskose Reaktionsmasse noch ausreichend zu durchmischen.

In der US-PS 3 151 136 wird ein Verfahren beschrieben, bei dem eine Hydroxyalkansulfonsäure mit einer Fettsäure verestert wird. Diese Reaktion kann in Gegenwart eines Lösungsmittels durchgeführt werden, wobei als Lösungsmittel die als Ausgangsprodukt eingesetzte Sulfonsäure oder das Veresterungsprodukt der Sulfonsäure mit der ebenfalls einzusetzenden Fettsäure verwendet werden kann.

Das in dieser US-Patentschrift beschriebene Verfahren ist umständlich, da zunächst die freie Hydroxyalkansulfonsäure hergestellt werden muß, indem man das entsprechende Natriumsalz zunächst mit Salzsäure oder gasförmigem Chlorwasserstoff behandeln muß; anschließend ist die Entfernung des entstandenen Kochsalzes erforderlich. Nach der Veresterung, d.h. nach der Kondensation muß die erhaltene freie Säure zusätzlich neutralisiert werden. Auch müssen die als Lösungsmittel eingesetzten Verbindungen, soweit sie nicht selbst das Veresterungsprodukt sind, wenigstens teilweise entfernt werden.

In der US-PS 3 880 897 wird empfohlen, die Herstellung der Acylester der Hydroxyalkansulfonate in Dialkylketonen mit 4 bis 8 Kohlenstoffatomen als Lösungsmittel durchzuführen.

Dieses Verfahren ist umständlich, außerdem muß das Keton nach der Umsetzung entfernt werden. Schließlich haften dem End produkt stets Reste von den eingesetzten Dialkylketonen an, die besonders bei Einsatzzwecken in der Kosmetik, wo es auf äußerst reine Produkte ankommt, von Nachteil sind.

In der US-PS 3 420 858 wird gelehrt, bei der Herstellung von Acylisoäthionaten Fettsäuren, insbesondere Stearinsäure, zuzumischen. Dieses Verfahren ist von Nachteil, da einerseits Stearoylisethionate als Nebenprodukt entstehen und zum anderen die vorhandene Stearinsäure im Endprodukt störend wirken kann. Die Abtrennung der Stearinsäure ist mit Schwierigkeiten verbunden.

Auch das in der US-PS 4 536 338 beschriebene Verfahren, bei dem der Veresterungskatalysator durch Zusatz von basischen Verbindungen vor der Zugabe von Stearinsäure desaktiviert wird, bringt hier nur wenig Abhilfe.

Gegenstand des Hauptpatents Nr. 3 442 579 ist ein Verfahren zur Herstellung von oberflächenaktiven Kondensationsprodukten durch Verestern von Carbonsäuren mit Salzen von Hydroxyalkansulfonsäuren, dadurch gekennzeichnet, daß man Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der allgemeinen

Formel HO-(CH2)n-SO3X, wobei n Werte von 2 bis 4 bedeutet und X ein Alkalimetall oder NH4 bedeutet, verestert, wobei man Ester der allgemeinen Formel R'COOR'', in der R' ein gesättigter und/oder ungesättigter Rest mit 7 bis 17 Kohlenstoffatomen und einer Menge von 1 bis 50 Gew.-%, bezogen auf die Menge der Ausgangsstoffe, als Konsistenzregler einsetzt.

Aufgabe der Erfindung ist es, das Verfahren gemäß Patent 3 442 579 zu verbessern und besser handhabbar zu machen und zu Produkten mit vorteilhaften Eigenschaften zu gelangen.

Diese Aufgabe wird gelöst durch ein Verfahren zur Herstellung von oberflächenaktiven Kondensationsprodukten durch Verestern von Carbonsäuren mit Salzen von Hydroxyalkansulfonsäuren, indem man Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der allgemeinen Formel HO-(CH2)n-SO3X, wobei n Werte von 2 bis 4 bedeuten und X ein Alkalimetall oder NH4 bedeutet, in Gegenwart eines Konsistenzreglers verestert, nach Patent 3 442 579, dadurch gekennzeichnet, daß man als Konsistenzregler bei Zimmertemperatur flüssige, halbfeste oder feste Paraffine verwendet.

Vorzugsweise werden Paraffine verwendet, die als Hauptbestandteil Kohlenwasserstoffe mit mindestens 16 C-Atomen aufweisen. Als Paraffine ist Mikrowachs besonders geeignet. Ebenfalls hervorragend geeignet ist raffiniertes Tafelparaffin.

Die Paraffine werden im allgemeinen in Mengen von 1,5 bis 100 %, vorzugsweise 1,5 bis 50 bzw. 1,5 - 15 Gew.-%, bezogen auf die als Ausgangsstoffe eingesetzten Carbonsäuren und Salze der Hydroxyalkansulfonsäuren verwendet.

Unter Paraffinen im Sinne der Erfindung sind übliche bei Zimmertemperatur flüssige halbfeste oder feste aliphatische Kohlenwasserstoffe zu verstehen, die natürlichen Ursprungs sein können oder auf synthetische Weise hergestellt sein können.

Paraffine, wie sie gemäß der Erfindung eingesetzt werden können, sind z.B. in Römpps-Chemie Lexikon 8. Auflage, Franckh'sche Verlagshandlung, Stuttgart genannt. Eine ausführlichere Beschreibung findet sich auf Seite 97-119 in "Laboratoriumsbuch für die Untersuchung technischer Wachs-, Harz-und Ölgemenge" von E.J. Fischer und Dr. W. Presting, VEB Wilhelm Knapp Verlag, Halle (Saale) 1958. Insbesondere die dort erwähnten Paraffinöle, Vaseline, Weich-und Hartparaffine sind geeignete Produkte. Es versteht sich von selbst, daß diese Paraffinprodukte vor allem, wenn die hergestellten Acylisethionate als Waschmittel oder in der Kosmetik eingesetzt werden sollen, vor Einsatz einer entsprechenden Raffination unterzogen werden. Derartige Reinigungsverfahren sind bekannt und können z.B. in einer einfachen Destillation bestehen, üblich sind auch Umkristallisierungsverfahren, Extraktionsverfahren oder Bleichen.

Weiter geeignet sind auch sogenannte "Hartparaffine", brauchbar sind auch Erdwachs (Ozokerit), das insbesondere in seiner farblosen Form als Ceresin geeignet ist.

Sehr geeignet sind auch Mikrowachse, wie sie z.B. in "Römpps Chemie-Lexikon" 7. Auflage, Franckh'sche Verlagshandlung, Stuttgart beschrieben werden.

Farblose und geruchsarme Paraffine werden bevorzugt.

Sehr vorteilhaft ist auch die Verwendung von Paraffingemischen.

Das Herstellungsverfahren der oberflächenaktiven Kondensationsprodukte durch Verestern von Carbonsäure mit Salzen von Hydroxyalkansulfonsäuren gemäß der Erfindung kann grundsätzlich auf die Art und Weise durchgeführt werden, wie es im Hauptpatent beschrieben wird, es wird lediglich anstelle der Ester mit der Formel R'COOR'' Paraffin eingesetzt. Die Paraffine können bereits von Anfang an den Ausgangsstoffen zugemischt werden, sie können aber auch im Verlauf der Umsetzung oder gegen Ende der Veresterung hinzugefügt werden. Auch eine portionsweise Zugabe während der Umsetzung ist möglich. Durch den Zusatz der Paraffine wird während der Veresterung nicht nur die Konsistenz des Reaktionsansatzes auf günstigen Werten gehalten sondern es ist auch nicht notwendig, die Paraffine nach Abschluß der Kondensation aus dem Reaktionsgemisch zu entfernen, da sie vor allem in Syndet-Stücken wertvolle Inhaltsstoffe sind, die im Endprodukt verbleiben können.

Von Vorteil ist ferner, daß bei der Destillation, durch die Wasser und freie Fettsäure entfernt werden, der Restgehalt an freier Fettsäure erheblich herabgesetzt werden kann, und zwar auf Säurezahlen von 25 bis 15 oder weniger. Dies ist von großem Vorteil, da in zahlreichen Anwendungsgebieten die Anteile von freien Fettsäuren, insbesondere von kurzkettigen, möglichst gering sein soll, zum Teil sogar völlig unerwünscht sind.

Der Restgehalt an freier Fettsäure läßt sich durch den Einsatz des Paraffins steuern. So ist es möglich, durch Erhöhung des Paraffingehaltes den Gehalt an Fettsäure auf die gewünschten niedrigen Restgehalte zu reduzieren.

Die gemäß der Erfindung erhaltenen Gemische von oberflächenaktiven Kondensationsprodukten und Paraffinen können direkt zu Syndet-Stücken verarbeitet werden. Gemäß dem Verfahren ist es möglich, den Paraffingehalt im Endprodukt durch Einstellung bestimmter Ausgangsmengen zu steuern, so daß man sehr vielseitig in der Herstellung von Syndet-Stücken ist und sich auf die jeweiligen Anwendungsgebiete, wo unterschiedliche Paraffingehalte gewünscht werden, einstellen kann.

Die erhaltenen Produkte lassen sich im übrigen sehr gut mit den in der Waschmittelindustrie, in der Seifenherstellung und in der Kosmetik üblichen Zuschlägen, Füllstoffen und Additiven, wie z.B. Diäthanolamiden, Fettalkoholsulfaten, Sulfoacetaten, Fettsäuren, Glycerinestern, Stärkeprodukten, Salzen, Wasser u.dgl. zu fertig konfektionierten Produkten verarbeiten.

Es war besonders überraschend, daß Paraffine der genannten Art die z.T. als Plastifikatoren bei der Formulierung und Herstellung von Syndets durch Vermischen von fertigen Komponenten üblich sind, auch hervorragende Prozeßhilfsmittel bei der chemischen Synthese der Kondensationsprodukte darstellen, die das chemische Verfahren vereinfachen und auch anderweitig verbessern, z.B. kostengünstiger und besser kontrollierbar machen. Darüber hinaus führt das beschriebene Syntheseverfahren zu Produkten mit verbesserter Zusammensetzung und damit zu verbesserten anwendungstechnischen Eigenschaften. Schon geringe Einsatzmengen von ca. 2 % an Paraffin-Kohlenwasserstoffen sind geeignet, die Produktschmelze trotz einer hohen Konzentration an Acylisethionat und niedrigem Fettsäuregehalt noch bis zu Temperaturen von ca. 200°C fließ-und rührfähig zu halten.

Bei Einsatz von raffinierten farblosen Paraffinen ist auch das erhaltene Endprodukt farblos, was besonders in der Kosmetik von großem Vorteil ist.

Die Erfindung wird durch folgende Beispiele näher erläutert:

Beispiel 1

Einsatz von 1,6 %Tafelparaffin (bezogen auf Ausgangsmenge Fettsäure und Natriumoxethansulfonat)

In einem 1 l-Dreihalskolben, ausgestattet mit Destillationsbrücke, Rührer und Gaseinleitungsrohr, werden 227,8 Teile Kokosfettsäure (SZ: 273), 125,8 Teile Natriumoxethansulfonat, 4,3 Teile Phosphorsäure und 5,7 Teile Paraffin unter N₂-Atmosphäre innerhalb von ca. 2 Stunden unter Rühren auf 235°C aufgeheizt. Man hält für 4 Stunden bei dieser Temperatur und destilliert das entstehende Reaktionswasser zusammen mit geringen Mengen co-destillierten Fettsäuren ab. Anschließend wird Vakuum angelegt und überschüssige Fettsäure abdestilliert bis eine viskose noch rührfähige Masse vorliegt. Das Vakuum wird mit N₂ aufgehoben und das Produkt unter Rühren langsam auf Raumtemperatur gebracht. Trotz hohen Gehaltes an Acylisethionat bleibt die Reaktionsmasse bis ca. 200°C rührfähig.

Kennzahlen:    WAS = 85,1 %
            SZ = 22,2
            Klettfarbe = 22

Beispiel 2

Einsatz von 4,7 % Paraffin (bezogen auf Ausgangsmenge Fettsäure und Natriumoxethansulfonat)

In einem Reaktor, wie in Beispiel 1, werden 211,8 Teile Kokosfettsäure (SZ: 270,8, 125,8 g Natriumoxethansulfonat, 3,9 Teile Phosphorsäure und 15,9 Teile Paraffin unter Rühren und N₂-Schutzgas innerhalb von ca. 2 Stunden auf 235°C aufgeheizt. Man hält für 4 Stunden bei dieser Temperatur, destilliert das Reaktionswasser ab und legt dann vorsichtig Vakuum an, um das Produkt durch Abdestillieren der überschüssigen Fettsäuren aufzukonzentrieren.

Bei einem Enddruck von 23 mbar und 225 - 230°C wird die Destillation beendet und das Vakuum mit N₂ aufgehoben. Das Reaktionsprodukt bleibt beim Abkühlen bis ca. 200°C rührfähig.

Kennzahlen:    SZ = 16,1
            WAS = 82 %
            Klettfarbe = 14
SZ bedeutet Säurezahl,
WAS waschaktive Substanz.

Die Klettfarbe wird bestimmt mit Hilfe eines Klett Summerson Photoelectric Colorimeter, Filter KS 42, 40 mm Glasküvetten. Hersteller des Geräts ist die Klett Manufacturing Co. Inc., New York/USA.

**Ansprüche**

1. Verfahren zur Herstellung von oberflächenaktiven Kondensationsprodukten durch Verestern von Carbonsäuren mit Salzen von Hydroxyalkansulfonsäuren, indem man Carbonsäuren der allgemeinen Formel RCOOH, wobei R ein gesättigter und/oder ungesättigter Kohlenwasserstoffrest mit 7 bis 31 Kohlenstoffatomen ist, mit einem Salz der allgemeinen Formel HO-(CH₂)ₙ-SO₃X, wobei n Werte von 2 bis 4 bedeuten und X ein Alkalimetall oder NH₄ bedeutet, in Gegenwart eines Konsistenzreglers verestert, nach Patent 3 442 579, dadurch gekennzeichnet, daß man als Konsistenzregler bei Zimmertemperatur flüssige, halbfeste oder feste Paraffine verwendet.

2. Verfahren nach Anspruch 1, dadurch gekennzeichnet, daß man Paraffine verwendet, deren Hauptbestandteil Kohlenwasserstoffe mit mindestens 16 C-Atomen sind.

3. Verfahren nach Anspruch 1, dadurch gekennzeichnet, daß man als Paraffin Mikrowachs verwendet.

4. Verfahren nach Anspruch 1, dadurch gekennzeichnet, daß man als Paraffin raffiniertes Tafelparaffin verwendet.

5. Verfahren nach einem der Ansprüche 1 bis 4, dadurch gekennzeichnet, daß man Paräffine in Mengen von 1,5 bis 100 %, bezogen auf die eingesetzte Menge Carbonsäure und Salze von Hydroxyalkansulfonsäuren, verwendet.

6. Verfahren nach Anspruch 5, dadurch gekennzeichnet, daß man 1,5 bis 50 Gew.-% Paraffine verwendet.

7. Verfahren nach Anspruch 6, dadurch gekennzeichnet, daß man 1,5 - 15 Gew.-% Paraffine verwendet.

8. Verfahren nach einem der Ansprüche 1 bis 7, dadurch gekennzeichnet, daß man Gemische von Paraffinen verwendet.

**Europäisches Patentamt**

**EUROPÄISCHER TEILRECHERCHENBERICHT,** der nach Regel 45 des Europäischen Patent- übereinkommens für das weitere Verfahren als europäischer Recherchenbericht gilt

Nummer der Anmeldung

EP 87 10 6230

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | US-A-3 320 292 (A. CAHN et al.) <br><br> * Column 2, line 62 - column 3, line 2 * <br><br>--- | | C 07 C 143/10 |
| A,D | US-A-3 151 136 (E. KOCZOROWSKI et al.) | | |
| P,A | DE-A-3 442 579 (AKZO) <br><br> --------------- | | |

**RECHERCHIERTE SACHGEBIETE (Int. Cl.4)**

C 07 C 143/00

## UNVOLLSTÄNDIGE RECHERCHE

Nach Auffassung der Recherchenabteilung entspricht die vorliegende europäische Patentanmel- dung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche:

Unvollständig recherchierte Patentansprüche:

Nicht recherchierte Patentansprüche:

Grund für die Beschränkung der Recherche:

Die Recherche ist nur dann sinnvoll, falls die in den Patentansprüchen und der Beschreibung erwähnte Patentschrift 3 442 579, auch tatsächlich die Deutsche Patentschrift 3 442 579 ist.

| Recherchenort | Abschlußdatum der Recherche | Prüfer |
|---|---|---|
| Den Haag | 31-07-1987 | VAN GEYT |